(19) Europäisches Patentamt
European Patent Office
Office européen des brevets

(11) **EP 4 432 166 A1**

(12) **EUROPEAN PATENT APPLICATION**

(43) Date of publication:
**18.09.2024 Bulletin 2024/38**

(21) Application number: **23465505.8**

(22) Date of filing: **15.03.2023**

(51) International Patent Classification (IPC):
**G06N 3/045** (2023.01)      **G06N 3/084** (2023.01)
**G06N 3/096** (2023.01)      **G06N 3/0464** (2023.01)

(52) Cooperative Patent Classification (CPC):
**G06N 3/096; G06N 3/045; G06N 3/084;**
G06N 3/0464; G06T 7/0012; G16H 30/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• GEORGESCU, Bogdan
  Princeton, NJ 08540 (US)
• SERBAN, Alexandru Constantin
  900520 Constanta (RO)
• GHESU, Florin-Cristian
  91054 Erlangen (DE)
• NEUMANN, Dominik
  91052 Erlangen (DE)
• NARASIMHAMURTHY, Venkatesh
  Hillsborough, NJ 08844 (US)
• GULSUN, Mehmet Akif
  Princeton, NJ 08540 (US)
• COMANICIU, Dorin
  Princeton, NJ 08540 (US)

(74) Representative: **Schwarz, Claudia**
**Schwarz + Kollegen**
**Patentanwälte**
**Heilmannstraße 19**
**81479 München (DE)**

(54) **INVARIANCE PRESERVING KNOWLEDGE DISTILLATION OF FOUNDATIONAL MODELS FOR MEDICAL IMAGING ANALYSIS**

(57)    Systems and methods for adapting a foundational model for performing a medical imaging analysis task are provided. An invariant based training data set is determined based on conditions on which a foundational model is invariant to one or more properties. The foundational model is adapted, based on the invariant based training data set, into an adapted model for performing the medical imaging analysis task. The adapting preserves the invariance to the one or more properties in the adapted model. The adapted model is output.

FIG. 2

EP 4 432 166 A1

**Description**

TECHNICAL FIELD

The present invention relates generally to artificial intelligence and machine learning, and in particular to invariance preserving knowledge distillation of foundational models for medical imaging analysis.

BACKGROUND

In artificial intelligence and machine learning, a foundational model is a model trained on large scale unlabeled data sets and can be adapted to perform a wide range of downstream tasks. During training, instead of learning to perform a particular downstream task, the foundation model learns to create complex representations and relationships, while preserving invariances to various properties. For example, the foundational model may be invariant to distribution shifts, geometrical transformations, contrast transformations, intensity transformations, etc. Once trained, the foundational model is adapted to perform a particular downstream task by distilling the foundational model into a smaller, more efficient model and by fine-tuning the distilled foundational model to perform the downstream task.

Conventionally, a foundational model is adapted to perform a particular downstream task by optimizing metrics related to the performance of the particular downstream task. However, such conventional approaches for adapting the foundational model do not consider other important properties of the foundational model, such as, e.g., the richness of representations or their invariance to various properties. Depending on the downstream task, it may be desirable to preserve invariances to certain properties while maintaining or improving performance of the foundational model. For example, preserving invariance to distribution shifts, geometrical transformations, or some intensity transformations may result in a more robust downstream adapted model if such variations are not well represented in the training data sets. Such conventional approaches do not preserve invariances when adapting the foundational model to perform the downstream task.

BRIEF SUMMARY OF THE INVENTION

In accordance with one or more embodiments, systems and methods for adapting a foundational model for performing a medical imaging analysis task are provided. An invariant based training data set is determined based on conditions on which a foundational model is invariant to one or more properties. The foundational model is adapted, based on the invariant based training data set, into an adapted model for performing the medical imaging analysis task. The adapting preserves the invariance to the one or more properties in the adapted model. The adapted model is the output.

In one embodiment, the foundational model is adapted into the adapted model based on an invariance preserving loss. The invariance preserving loss is based on a similarity between non-invariant samples and invariant samples in the invariant based training data set. In one embodiment, the foundational model is further adapted into the adapted model based on a distillation loss. A loss aggregation mechanism moderates the contribution of the invariance preserving loss and the distillation loss.

In one embodiment, the invariant based training data set is determined by selecting an existing training data set comprising data that satisfies the conditions on which the foundational model is invariant to the one or more properties. In another embodiment, the invariant based training data set is determined by applying one or more transformations on samples of a distillation data set to generate transformed samples that satisfy the conditions on which the foundational model is invariant to the one or more properties.

In one embodiment, the one or more properties are determined based on the medical imaging analysis task. The one or more properties may comprise at least one of distribution, geometric, contrast level, or intensity level.

In one embodiment, the medical imaging analysis task comprises at least one of classification, detection, or segmentation.

In accordance with one embodiments, systems and methods for performing a medical imaging analysis task are provided. One or more input medical images are received. A medical imaging analysis task is performed on the one or more input medical images using an adapted model. Results of the medical imaging analysis task are output. The adapted model is adapted from a foundational model by determining an invariant based training data set based on conditions on which the foundational model is invariant to one or more properties and adapting, based on the invariant based training data set, the foundational model into the adapted model for performing the medical imaging analysis task. The adapting preserves the invariance to the one or more properties for the adapted model.

These and other advantages of the invention will be apparent to those of ordinary skill in the art by reference to the following detailed description and the accompanying drawings.

BRIEF DESCRIPTION OF THE DRAWINGS

Figure 1 shows a method for adapting a foundational model to perform a medical imaging analysis task, in accordance with one or more embodiments;
Figure 2 shows a workflow for adapting a foundational model to perform a medical imaging analysis task, in accordance with one or more embodiments;
Figure 3 shows a method for performing a medical imaging analysis task using an adapted model; in accordance with one or more embodiments;
Figure 4 shows an exemplary artificial neural network that may be used to implement one or more embodiments;
Figure 5 shows a convolutional neural network that may be used to implement one or more embodiments; and
Figure 6 shows a high-level block diagram of a computer that may be used to implement one or more embodiments.

DETAILED DESCRIPTION

The present invention generally relates to methods and systems for invariance preserving knowledge distillation of foundational models for medical imaging analysis. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Foundational models are trained on large scale unlabeled training data sets and are typically invariant to certain properties, such as, e.g., distribution shifts, geometrical transformations, contrast transformations, intensity transformations, etc. Embodiments described herein provide for the adaptation of foundational models for performing a downstream medical imaging analysis task while preserving invariances to one or more properties. The adaptation of foundational models is performed in accordance with one or more embodiments described herein by identifying conditions on which the foundational model is invariant to the one or more properties based on the downstream medical imaging analysis task, by determining an invariant based training data set based on the identified conditions, and/or by adapting, based on the invariant based training data set, the foundational model to perform the downstream medical imaging analysis task based on an invariance preserving loss function. Advantageously, foundational models are adapted in accordance with embodiments described herein to preserve invariance to certain properties to thereby improve the representation power of the adapted models and, inherently, their robustness and performance.

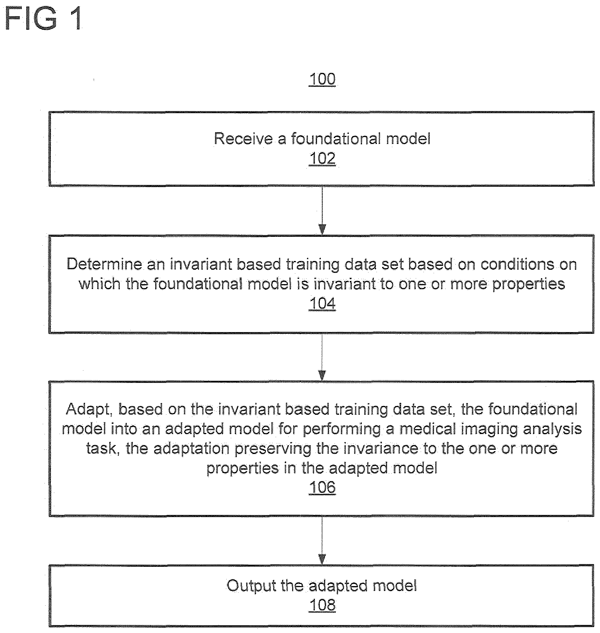

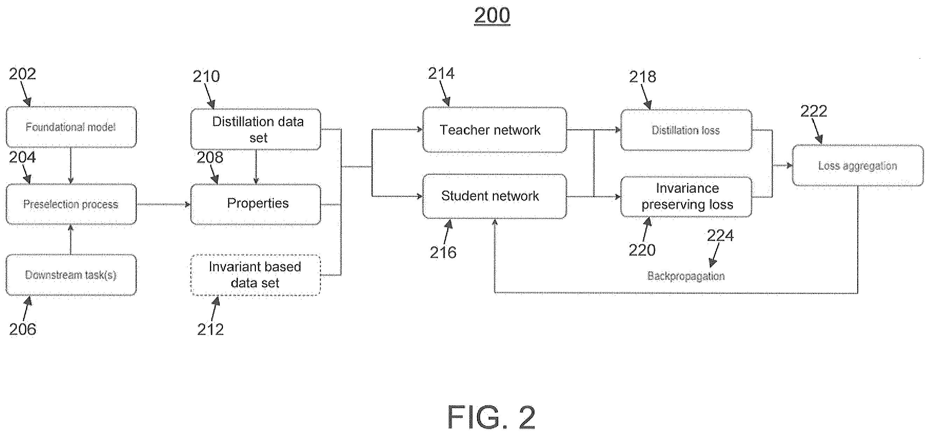

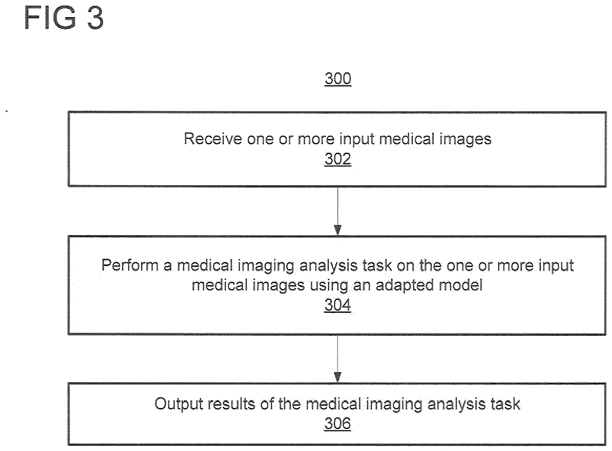

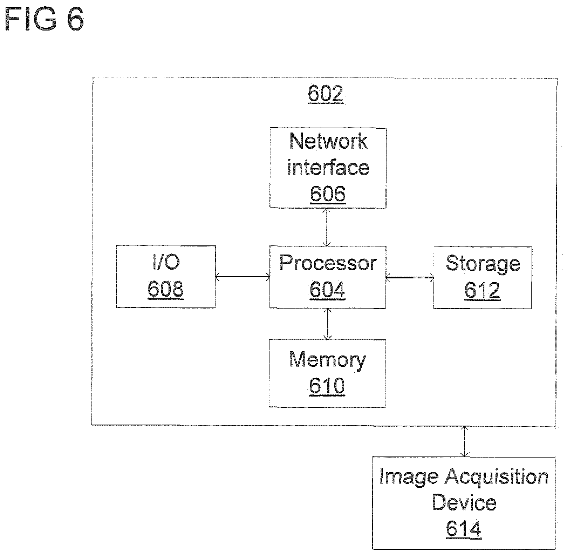

Figure 1 shows a method 100 for adapting a foundational model to perform a medical imaging analysis task, in accordance with one or more embodiments. Figure 2 shows a workflow 200 for adapting a foundational model to perform a medical imaging analysis task, in accordance with one or more embodiments. Figure 1 and Figure 2 will be described together. The steps of method 100 may be performed by one or more suitable computing devices, such as, e.g., computer 602 of Figure 6. Method 100 of Figure 1 and workflow 200 of Figure 2 are performed during an offline or training stage for training an adapted model for performing a medical imaging analysis task. Once trained, the adapted model may be applied to perform the medical imaging analysis task during an online or inference stage, for example, according to method 300 of Figure 3.

At step 102 of Figure 1, a foundational model is received. In one example, as shown in workflow 200 of Figure 2, the foundational model is foundational model 202. Foundational model 202 is trained on broad and large scale unlabeled training data sets and is invariant to various properties. For example, foundational model 202 may be invariant to distribution shifts, geometrical transformations, contrast level transformations, intensity level transformations, etc. Foundational model 202 is invariant to various properties such that performance of foundational model 202 is generally consistent on transformations of such properties on input data. Foundational model 202 may be received by, for example, loading foundational model 202 from a storage or memory of a computer system or receiving foundational model 202 from a remote computer system.

At step 104 of Figure 1, an invariant based training data set is determined based on conditions on which the foundational model is invariant to one or more properties. In one embodiment, the invariant based training data set is a training data set that satisfies the conditions on which the foundational model is invariant to the one or more properties. For example, the invariant based training data set may comprise training images with different contrast levels that are within the conditions on which the foundational model is invariant to the contrast levels.

To determine the invariant based training data set, the one or more properties that the foundational model is invariant to are first selected based on the downstream medical imaging analysis task that the foundational model will be adapted to perform (at step 106 of Figure 1). In one example, as shown in workflow 200 of Figure 2, one or more properties 208 that foundational model 202 is invariant to are selected in a preselection process 204 based on downstream task(s) 206. Downstream tasks 206 may comprise any suitable medical imaging analysis task, such as, e.g., classification, detection, segmentation, etc. For example, during preselection process 204, where downstream task 206 comprises detection of anatomical objects (e.g., organs, bones, vessels, lesions, abnormalities, etc.) in medical images and such medical images are acquired at different contrast levels according to the image acquisition process, the invariance of foundational model 202 on the different contrast levels is tested on a subset of a distillation data set 210 for adapting foundational model 202 to perform downstream task 206. The contrast property is selected in response to the testing confirming that foundational model 202 is invariant to the different contrast levels and the conditions (or bounds) on which foundational model 202 is invariant to the different contrast levels are determined. Such conditions will help to determine the invariant based training data set (at step 104 of Figure 1) and an invariance preserving loss function for adapting the foundational model to perform the downstream medical imaging analysis task (at step 106 of Figure 1).

Invariant based training data set 212 is then determined based on the conditions on which foundational model 202 is invariant to the one or more properties (selected during preselection process 204). In one embodiment, invariant based training data set 212 is determined by selecting an existing training data set comprising data that satisfies the conditions on which foundational model 202 is invariant to the one or more properties. In another embodiment, for example where such an existing training data set is not available, invariant based training data set 212 is generated by transforming distillation data set 210 to satisfy the conditions on which foundational model 202 is invariant to the one or more properties. Distillation data set 210 may be transformed by applying one or more transformations to samples of distillation data set 210 to generate transformed samples that satisfy the conditions on which foundational model 202 is invariant to the one or more properties. Examples of such transformations include rotation, translation, cropping, flipping, scaling, adding noise, adjusting contrast, etc. Such transformations generate data that can introduce high uncertainty when adapting foundational model 202 (at step 106 of Figure 1).

At step 106 of Figure 1, the foundational model is adapted, based on the invariant based training data set, into an adapted model for performing a medical imaging analysis task. The adaptation preserves the invariance to the one or more properties in the adapted model.

In one embodiment, the foundational model is adapted (e.g., distilled and/or fine-tuned) into the adapted model based on student-teacher knowledge distillation. For example, workflow 200 of Figure 2 shows a teacher network 214 representing the relatively larger foundational model 202 and a student network 216 representing the relatively smaller adapted model. Student network 216 learns to mimic the output of teacher network 214 such that knowledge of teacher network 214 is distilled or transferred to student network 216. Student network 216 is less computationally expensive while maintaining or improving the performance of teacher network 214 and thus student network 216 is more suitable for deployment.

Student-teach knowledge distillation is performed according to an invariance preserving loss 220. invariance preserving loss 216 conditions the distillation on the invariance to the one or more properties from teacher network 214 to student network 216 while maintaining richness of representation. In one embodiment, invariance preserving loss 220 estimates the similarity between representations of normal samples (i.e., non-invariant samples that satisfy the conditions on which foundational model 202 is invariant to the one or more properties) and representations of invariant samples (that do not satisfy the conditions on which foundational model 202 is invariant to the one or more properties) in the invariant based training data set. By minimizing such similarity, invariance preserving loss 216 minimizes the difference between the representations of the normal and invariant samples. Invariance preserving loss 220 may be any suitable loss function for distilling the invariance to the one or more properties from teacher network 214 to student network 216. Invariance preserving loss 220 may be selected based on the one or more properties. Invariance preserving loss 220 may also be a generally defined loss such as, e.g., a measure of the batch-wise Kullback-Leibler divergence between the likelihoods (e.g., output of a neural network) of the normal samples and their specific invariants.

Invariance preserving loss 216 may be used together with a distillation loss 218 to preserve or distill the richness of representations learned by teacher network 214. In one example, distillation loss 218 is the Kullback-Leibler divergence loss. However, distillation loss 218 may be any other suitable loss for distilling knowledge from teacher network 214 to student network 216. The contribution of distillation loss 218 and invariance preserving loss 220 may be moderated by loss aggregation mechanism 222 during the training via backpropagation 224.

At step 108 of Figure 1, the adapted model is output. In one example, as shown in workflow 200 of Figure 2, student network 216 represents the adapted model and student network 216 is output. The adapted model may be output by, for example, storing the adapted model on a memory or storage of a computer system or by transmitting the adapted model to a remote computer system. In one embodiment, the adapted model is output by deploying the adapted model to perform the medical imaging analysis task.

Advantageously, embodiments described herein preserve invariances of foundational models to one or more properties during distillation, thereby improving the representation power of the adapted model and, inherently, its robustness and performance. Further, embodiments described herein significantly reduce the need for data augmentation of the distillation data set used for adapting foundational models, as foundational models are typically trained with strong data augmentation and distilling this knowledge attenuates the need for further data augmentation of the distillation data set.

Figure 3 shows a method 300 for performing a medical imaging analysis task using an adapted model, in accordance with one or more embodiments. The steps of method 300 may be performed by one or more suitable computing devices, such as, e.g., computer 702 of Figure 7. Method 300 is performed using the trained adapted model during an online or inference stage.

At step 302 of Figure 3, one or more input medical images are received. The one or more input medical images may depict anatomical objects (e.g., organs, bones, vessels, lesions, abnormalities, etc.) of a patient. The one or more input medical images may be of any suitable modality, such as, e.g., CT (computed tomography), MRI (magnetic resonance imaging), ultrasound, x-ray, or any other medical imaging modality or combinations of medical imaging modalities, and may be 2D (two dimensional) images and/or 3D (three dimensional) volumes. The one or more input medical images may be received directly from an image acquisition device, such as, e.g., a CT scanner, as the medical images are acquired, or can be received by loading previously acquired medical images from a storage or memory of a computer system or receiving medical images that have been transmitted from a remote computer system.

At step 304 of Figure 3, a medical imaging analysis task is performed on the one or more input medical images using an adapted model. The medical imaging analysis task may comprise suitable medical imaging analysis task performed using the adapted model, such as, e.g., classification, detection, segmentation, etc. In one embodiment, the adapted model is adapted from a foundational model during a prior offline or training stage, for example, in accordance with method 100 of Figure 1 and workflow 200 of Figure 2.

At step 306 of Figure 3, results of the medical imaging analysis task are output. For example, the results of the medical imaging analysis task can be output by displaying the results of the medical imaging analysis task on a display device of a computer system, storing the results of the medical imaging analysis task on a memory or storage of a computer system, or by transmitting the results of the medical imaging analysis task to a remote computer system.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based models, as well as with respect to methods and systems for training machine learning based models. Features, advantages or alternative embodiments herein can be assigned to the other claimed objects and vice versa. In other words, claims for methods and systems for training a machine learning based model can be improved with features described or claimed in context of the methods and systems for utilizing a trained machine learning based model, and vice versa.

In particular, the trained machine learning based models applied in embodiments described herein can be adapted by the methods and systems for training the machine learning based models. Furthermore, the input data of the trained machine learning based model can comprise advantageous features and embodiments of the training input data, and vice versa. Furthermore, the output data of the trained machine learning based model can comprise advantageous features and embodiments of the output training data, and vice versa.

In general, a trained machine learning based model mimics cognitive functions that humans associate with other human minds. In particular, by training based on training data, the trained machine learning based model is able to adapt to new circumstances and to detect and extrapolate patterns.

In general, parameters of a machine learning based model can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based model can be adapted iteratively by several steps of training.

In particular, a trained machine learning based model can comprise, for example, a neural network, a support vector machine, a decision tree, and/or a Bayesian network, and/or the trained machine learning based network can be based on k-means clustering, Q-learning, genetic algorithms, and/or association rules. In particular, a neural network can be, for example, a deep neural network, a convolutional neural network, or a convolutional deep neural network. Furthermore, a neural network can be, for example, an adversarial network, a deep adversarial network and/or a generative adversarial network.

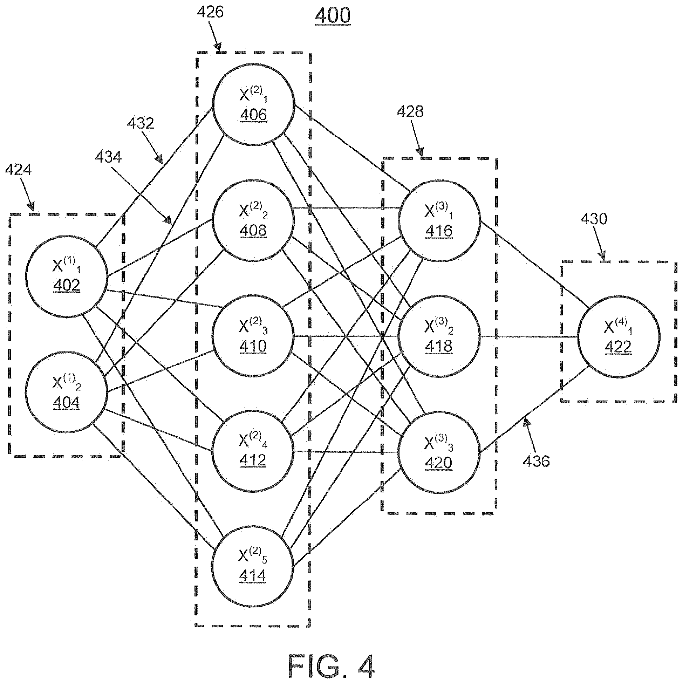

Figure 4 shows an embodiment of an artificial neural network 400, in accordance with one or more embodiments. Alternative terms for "artificial neural network" are "neural network", "artificial neural net" or "neural net". Machine learning networks described herein, such as, e.g., the foundational model and the adapted model utilized in method 100 of Figure 1, foundational model 202, teacher network 214, and student network 216 of workflow 200 of Figure 2, and the adapted model and the foundational model utilized in method 300 of Figure 3, may be implemented using artificial neural network 400.

The artificial neural network 400 shown in Figure 4 is a feedforward neural network comprising nodes 402-422 and edges 432, 434, ..., 436, wherein each edge 432, 434, ..., 436 is a directed connection from a first node 402-422 to a second node 402-422. In general, the first node 402-422 and the second node 402-422 are different nodes 402-422, it is also possible that the first node 402-422 and the second node 402-422 are identical. For example, in Figure 4, the edge 432 is a directed connection from the node 402 to the node 406, and the edge 434 is a directed connection from the node 404 to the node 406. An edge 432, 434, ..., 436 from a first node 402-422 to a second node 402-422 is also denoted as "ingoing edge" for the second node 402-422 and as "outgoing edge" for the first node 402-422.

In this embodiment, the nodes 402-422 of the artificial neural network 400 can be arranged in layers 424-430, wherein the layers can comprise an intrinsic order introduced by the edges 432, 434, ..., 436 between the nodes 402-422. In particular, edges 432, 434, ..., 436 can exist only between neighboring layers of nodes. In the embodiment shown in Figure 4, there is an input layer 424 comprising only nodes 402 and 404 without an incoming edge, an output layer 430 comprising only node 422 without outgoing edges, and hidden layers 426, 428 in-between the input layer 424 and the output layer 430. In general, the number of hidden layers 426, 428 can be chosen arbitrarily. The number of nodes 402 and 404 within the input layer 424 usually relates to the number of input values of the neural network 400, and the number of nodes 422 within the output layer 430 usually relates to the number of output values of the neural network 400.

In particular, a (real) number can be assigned as a value to every node 402-422 of the neural network 400. Here, $x^{(n)}_i$ denotes the value of the i-th node 402-422 of the n-th layer 424-430. The values of the nodes 402-422 of the input layer 424 are equivalent to the input values of the neural network 400, the value of the node 422 of the output layer 430 is equivalent to the output value of the neural network 400. Furthermore, each edge 432, 434, ..., 436 can comprise a weight being a real number, in particular, the weight is a real number within the interval [-1, 1] or within the interval [0, 1]. Here, $w^{(m,n)}_{i,j}$ denotes the weight of the edge between the i-th node 402-422 of the m-th layer 424-430 and the j-th node 402-422 of the n-th layer 424-430. Furthermore, the abbreviation $w^{(n)}_{i,j}$ is defined for the weight $w^{(n,n+1)}_{i,j}$.

In particular, to calculate the output values of the neural network 400, the input values are propagated through the neural network. In particular, the values of the nodes 402-422 of the (n+1)-th layer 424-430 can be calculated based on the values of the nodes 402-422 of the n-th layer 424-430 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smooth step function) or rectifier functions. The transfer function is mainly used for normalization purposes.

In particular, the values are propagated layer-wise through the neural network, wherein values of the input layer 424 are given by the input of the neural network 400, wherein values of the first hidden layer 426 can be calculated based on the values of the input layer 424 of the neural network, wherein values of the second hidden layer 428 can be calculated based in the values of the first hidden layer 426, etc.

In order to set the values $w^{(m,n)}_{i,j}$ for the edges, the neural network 400 has to be trained using training data. In particular, training data comprises training input data and training output data (denoted as $t_i$). For a training step, the neural network 400 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 400 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta_j^{(n)} = \left( x_k^{(n+1)} - t_j^{(n+1)} \right) \cdot f' \left( \sum_i x_i^{(n)} \cdot w_{i,j}^{(n)} \right)$$

if the (n+1)-th layer is the output layer 430, wherein f' is the first derivative of the activation function, and $y^{(n+1)}_j$ is the comparison training value for the j-th node of the output layer 430.

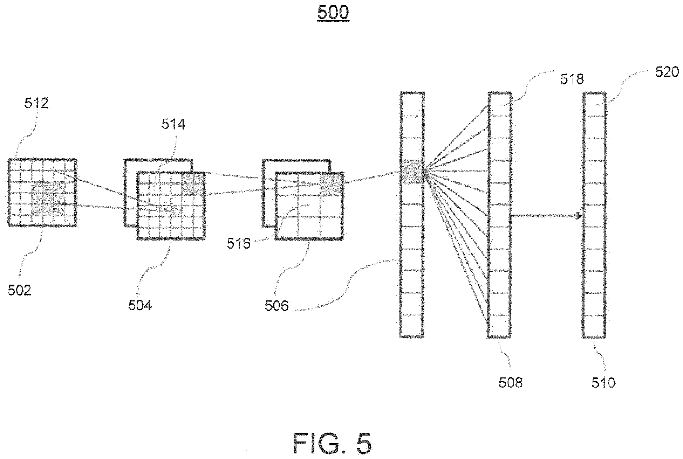

Figure 5 shows a convolutional neural network 500, in accordance with one or more embodiments. Machine learning networks described herein, such as, e.g., the foundational model and the adapted model utilized in method 100 of Figure 1, foundational model 202, teacher network 214, and student network 216 of workflow 200 of Figure 2, and the adapted model and the foundational model utilized in method 300 of Figure 3, may be implemented using convolutional neural network 500.

In the embodiment shown in Figure 5, the convolutional neural network comprises 500 an input layer 502, a convolutional layer 504, a pooling layer 506, a fully connected layer 508, and an output layer 510. Alternatively, the convolutional neural network 500 can comprise several convolutional layers 504, several pooling layers 506, and several fully connected layers 508, as well as other types of layers. The order of the layers can be chosen arbitrarily, usually fully connected layers 508 are used as the last layers before the output layer 510.

In particular, within a convolutional neural network 500, the nodes 512-520 of one layer 502-510 can be considered to be arranged as a d-dimensional matrix or as a d-dimensional image. In particular, in the two-dimensional case the value of the node 512-520 indexed with i and j in the n-th layer 502-510 can be denoted as $x^{(n)}_{[i,j]}$. However, the arrangement of the nodes 512-520 of one layer 502-510 does not have an effect on the calculations executed within the convolutional neural network 500 as such, since these are given solely by the structure and the weights of the edges.

In particular, a convolutional layer 504 is characterized by the structure and the weights of the incoming edges forming a convolution operation based on a certain number of kernels. In particular, the structure and the weights of the incoming edges are chosen such that the values $x^{(n)}_k$ of the nodes 514 of the convolutional layer 504 are calculated as a convolution $x^{(n)}_k = K_k * x^{(n-1)}$ based on the values $x^{(n-1)}$ of the nodes 512 of the preceding layer 502, where the convolution * is defined in the two-dimensional case as

$$x_k^{(n)}[i,j] = \left( K_k * x^{(n-1)} \right)[i,j] = \sum_{i'} \sum_{j'} K_k[i',j'] \cdot x^{(n-1)}[i-i', j-j'] .$$

Here the k-th kernel $K_k$ is a d-dimensional matrix (in this embodiment a two-dimensional matrix), which is usually small compared to the number of nodes 512-518 (e.g. a 3x3 matrix, or a $5 \times 5$ matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 512-520 in the respective layer 502-510. In particular, for a convolutional layer 504, the number of nodes 514 in the convolutional layer is equivalent to the number of nodes 512 in the preceding layer 502 multiplied with the number of kernels.

If the nodes 512 of the preceding layer 502 are arranged as a d-dimensional matrix, using a plurality of kernels can be interpreted as adding a further dimension (denoted as "depth" dimension), so that the nodes 514 of the convolutional layer 504 are arranged as a (d+1)-dimensional matrix. If the nodes 512 of the preceding layer 502 are already arranged as a (d+1)-dimensional matrix comprising a depth dimension, using a plurality of kernels can be interpreted as expanding along the depth dimension, so that the nodes 514 of the convolutional layer 504 are arranged also as a (d+1)-dimensional matrix, wherein the size of the (d+1)-dimensional matrix with respect to the depth dimension is by a factor of the number of kernels larger than in the preceding layer 502.

The advantage of using convolutional layers 504 is that spatially local correlation of the input data can exploited by enforcing a local connectivity pattern between nodes of adjacent layers, in particular by each node being connected to only a small region of the nodes of the preceding layer.

In embodiment shown in Figure 5, the input layer 502 comprises 36 nodes 512, arranged as a two-dimensional 6x6 matrix. The convolutional layer 504 comprises 72 nodes 514, arranged as two two-dimensional 6x6 matrices, each of the two matrices being the result of a convolution of the values of the input layer with a kernel. Equivalently, the nodes 514 of the convolutional layer 504 can be interpreted as arranges as a three-dimensional 6x6x2 matrix, wherein the last dimension is the depth dimension.

A pooling layer 506 can be characterized by the structure and the weights of the incoming edges and the activation function of its nodes 516 forming a pooling operation based on a non-linear pooling function f. For example, in the two dimensional case the values $x^{(n)}$ of the nodes 516 of the pooling layer 506 can be calculated based on the values $x^{(n-1)}$ of the nodes 514 of the preceding layer 504 as

$$x^{(n)}[i,j] = f(x^{(n-1)}[id_1, jd_2], ..., x^{(n-1)}[id_1+d_1-1, jd_2+d_2-1])$$

In other words, by using a pooling layer 506, the number of nodes 514, 516 can be reduced, by replacing a number $d_1 \cdot d_2$ of neighboring nodes 514 in the preceding layer 504 with a single node 516 being calculated as a function of the values of said number of neighboring nodes in the pooling layer. In particular, the pooling function f can be the max-function, the average or the L2-Norm. In particular, for a pooling layer 506 the weights of the incoming edges are fixed and are not modified by training.

The advantage of using a pooling layer 506 is that the number of nodes 514, 516 and the number of parameters is reduced. This leads to the amount of computation in the network being reduced and to a control of overfitting.

In the embodiment shown in Figure 5, the pooling layer 506 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 508 can be characterized by the fact that a majority, in particular, all edges between nodes 516 of the previous layer 506 and the nodes 518 of the fully-connected layer 508 are present, and wherein the weight of each of the edges can be adjusted individually.

In this embodiment, the nodes 516 of the preceding layer 506 of the fully-connected layer 508 are displayed both as two-dimensional matrices, and additionally as non-related nodes (indicated as a line of nodes, wherein the number of nodes was reduced for a better presentability). In this embodiment, the number of nodes 518 in the fully connected layer 508 is equal to the number of nodes 516 in the preceding layer 506. Alternatively, the number of nodes 516, 518 can differ.

Furthermore, in this embodiment, the values of the nodes 520 of the output layer 510 are determined by applying the Softmax function onto the values of the nodes 518 of the preceding layer 508. By applying the Softmax function, the sum the values of all nodes 520 of the output layer 510 is 1, and all values of all nodes 520 of the output layer are real numbers between 0 and 1.

A convolutional neural network 500 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 500 can be trained based on the backpropagation algorithm. For preventing overfitting, methods of regularization can be used, e.g. dropout of nodes 512-520, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another data sets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-3. Certain steps or functions of the methods and workflows described herein, including one or more of the steps or functions of Figures 1-3, may be performed by a server or by another processor in a network-based cloud-computing system. Certain steps or functions of the methods and workflows described herein, including one or more of the steps of Figures 1-3, may be performed by a client computer in a network-based cloud computing system. The steps or functions of the methods and workflows described herein, including one or more of the steps of Figures 1-3, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-3, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 602 that may be used to implement systems, apparatus, and methods described herein is depicted in Figure 6. Computer 602 includes a processor 604 operatively coupled to a data storage device 612 and a memory 610. Processor 604 controls the overall operation of computer 602 by executing computer program instructions that define such operations. The computer program instructions may be stored in data storage device 612, or other computer readable medium, and loaded into memory 610 when execution of the computer program instructions is desired. Thus, the method and workflow steps or functions of Figures 1-3 can be defined by the computer program instructions stored in memory 610 and/or data storage device 612 and controlled by processor 604 executing the computer program instructions. For example, the computer program instructions can be implemented as computer executable code programmed by one skilled in the art to perform the method and workflow steps or functions of Figures 1-3. Accordingly, by executing the computer program instructions, the processor 604 executes the method and workflow steps or functions of Figures 1-3. Computer 602 may also include one or more network interfaces 606 for communicating with other devices via a network. Computer 602 may also include one or more input/output devices 608 that enable user interaction with computer 602 (e.g., display, keyboard, mouse, speakers, buttons, etc.).

Processor 604 may include both general and special purpose microprocessors, and may be the sole processor or one of multiple processors of computer 602. Processor 604 may include one or more central processing units (CPUs), for example. Processor 604, data storage device 612, and/or memory 610 may include, be supplemented by, or incorporated in, one or more application-specific integrated circuits (ASICs) and/or one or more field programmable gate arrays (FPGAs).

Data storage device 612 and memory 610 each include a tangible non-transitory computer readable storage medium. Data storage device 612, and memory 610, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 608 may include peripherals, such as a printer, scanner, display screen, etc. For example, input/output devices 608 may include a display device such as a cathode ray tube (CRT) or liquid crystal display (LCD) monitor for displaying information to the user, a keyboard, and a pointing device such as a mouse or a trackball by which the user can provide input to computer 602.

An image acquisition device 614 can be connected to the computer 602 to input image data (e.g., medical images) to the computer 602. It is possible to implement the image acquisition device 614 and the computer 602 as one device. It is also possible that the image acquisition device 614 and the computer 602 communicate wirelessly through a network. In a possible embodiment, the computer 602 can be located remotely with respect to the image acquisition device 614.

Any or all of the systems and apparatus discussed herein may be implemented using one or more computers such as computer 602.

One skilled in the art will recognize that an implementation of an actual computer or computer system may have other structures and may contain other components as well, and that Figure 6 is a high level representation of some of the components of such a computer for illustrative purposes.

independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

**Claims**

1. A computer-implemented method comprising:

   determining (104) an invariant based training data set (212) based on conditions on which a foundational model (202) is invariant to one or more properties (208);
   adapting (106), based on the invariant based training data set, the foundational model into an adapted model (216) for performing a medical imaging analysis task, the adapting preserving the invariance to the one or more properties in the adapted model; and
   outputting (108) the adapted model.

2. The computer-implemented method of claim 1, wherein adapting, based on the invariant based training data set, the foundational model into an adapted model for performing a medical imaging analysis task comprises:
   adapting the foundational model into the adapted model based on an invariance preserving loss (220).

3. The computer-implemented method of claim 2, wherein the invariance preserving loss is based on a similarity between non-invariant samples and invariant samples in the invariant based training data set.

4. The computer-implemented method of claim 2, wherein adapting, based on the invariant based training data set, the foundational model into an adapted model for performing a medical imaging analysis task further comprises:
   adapting the foundational model into the adapted model based on a distillation loss (218), wherein a loss aggregation mechanism (222) moderates contribution of the invariance preserving loss and the distillation loss.

5. The computer-implemented method of claim 1, wherein determining an invariant based training data set based on conditions on which a foundational model is invariant to one or more properties comprises:
   selecting an existing training data set comprising data that satisfies the conditions on which the foundational model is invariant to the one or more properties.

6. The computer-implemented method of claim 1, wherein determining an invariant based training data set based on conditions on which a foundational model is invariant to one or more properties comprises:
   applying one or more transformations on samples of a distillation data set (210) to generate transformed samples that satisfy the conditions on which the foundational model is invariant to the one or more properties.

7. The computer-implemented method of claim 1, wherein the one or more properties are determined based on the medical imaging analysis task (206).

8. The computer-implemented method of claim 1, wherein the one or more properties comprise at least one of distribution, geometric, contrast level, or intensity level.

9. The computer-implemented method of claim 1, wherein the medical imaging analysis task comprises at least one of classification, detection, or segmentation.

10. An apparatus comprising:

    means for determining (104) an invariant based training data set (212) based on conditions on which a foundational model (202) is invariant to one or more properties (208);
    means for adapting (106), based on the invariant based training data set, the foundational model into an adapted model (216) for performing a medical imaging analysis task, the adapting preserving the invariance to the one or more properties in the adapted model; and
    means for outputting (108) the adapted model.

11. The apparatus of claim 10, wherein the means for adapting, based on the invariant based training data set, the foundational model into an adapted model for performing a medical imaging analysis task comprises:
means for adapting the foundational model into the adapted model based on an invariance preserving loss (220).

12. The apparatus of claim 11, wherein the invariance preserving loss is based on a similarity between non-invariant samples and invariant samples in the invariant based training data set.

13. The apparatus of claim 11, wherein the means for adapting, based on the invariant based training data set, the foundational model into an adapted model for performing a medical imaging analysis task further comprises:
means for adapting the foundational model into the adapted model based on a distillation loss (218), wherein a loss aggregation mechanism (222) moderates contribution of the invariance preserving loss and the distillation loss.

14. The apparatus of claim 10, wherein the means for determining an invariant based training data set based on conditions on which a foundational model is invariant to one or more properties comprises:
means for selecting an existing training data set comprising data that satisfies the conditions on which the foundational model is invariant to the one or more properties.

15. A non-transitory computer readable medium storing computer program instructions, the computer program instructions when executed by a processor cause the processor to perform operations comprising:

determining (104) an invariant based training data set (212) based on conditions on which a foundational model (202) is invariant to one or more properties (208);
adapting (106), based on the invariant based training data set, the foundational model into an adapted model (216) for performing a medical imaging analysis task, the adapting preserving the invariance to the one or more properties in the adapted model; and
outputting (108) the adapted model.

16. The non-transitory computer readable medium of claim 15, wherein determining an invariant based training data set based on conditions on which a foundational model is invariant to one or more properties comprises:
applying one or more transformations on samples of a distillation data set (210) to generate transformed samples that satisfy the conditions on which the foundational model is invariant to the one or more properties.

17. The non-transitory computer readable medium of claim 15, wherein the one or more properties are determined based on the medical imaging analysis task (206).

18. The non-transitory computer readable medium of claim 15, wherein the one or more properties comprise at least one of distribution, geometric, contrast level, or intensity level.

19. The non-transitory computer readable medium of claim 15, wherein the medical imaging analysis task comprises at least one of classification, detection, or segmentation.

20. A computer-implemented method comprising:

receiving (302) one or more input medical images;
performing (304) a medical imaging analysis task on the one or more input medical images using an adapted model (216); and
outputting (306) results of the medical imaging analysis task,
wherein the adapted model is adapted from a foundational model (202) by:

determining (104) an invariant based training data set (212) based on conditions on which the foundational model is invariant to one or more properties (208), and
adapting (106), based on the invariant based training data set, the foundational model into the adapted model for performing the medical imaging analysis task, the adapting preserving the invariance to the one or more properties in the adapted model.

# FIG 1

100

```
┌─────────────────────────────────────────────────────────────┐
│                                                             │
│              Receive a foundational model                    │
│                          102                                 │
│                                                             │
└─────────────────────────────────────────────────────────────┘
                              │
                              ▼
┌─────────────────────────────────────────────────────────────┐
│                                                             │
│   Determine an invariant based training data set based on    │
│   conditions on which the foundational model is invariant    │
│          to one or more properties                           │
│                          104                                 │
│                                                             │
└─────────────────────────────────────────────────────────────┘
                              │
                              ▼
┌─────────────────────────────────────────────────────────────┐
│                                                             │
│   Adapt, based on the invariant based training data set,     │
│   the foundational model into an adapted model for           │
│   performing a medical imaging analysis task, the            │
│   adaptation preserving the invariance to the one or more    │
│          properties in the adapted model                     │
│                          106                                 │
│                                                             │
└─────────────────────────────────────────────────────────────┘
                              │
                              ▼
┌─────────────────────────────────────────────────────────────┐
│                                                             │
│               Output the adapted model                       │
│                          108                                 │
│                                                             │
└─────────────────────────────────────────────────────────────┘
```

## 200

FIG. 2

# FIG 3

300

```
Receive one or more input medical images
                  302
```

```
Perform a medical imaging analysis task on the one or more input
          medical images using an adapted model
                        304
```

```
Output results of the medical imaging analysis task
                      306
```

FIG. 4

500

FIG. 5

# FIG 6

**602**

Network interface **606**

I/O **608**

Processor **604**

Storage **612**

Memory **610**

Image Acquisition Device **614**

**EP 4 432 166 A1**

EUROPEAN SEARCH REPORT

Application Number

EP 23 46 5505

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GHESU FLORIN C ET AL: "Contrastive self-supervised learning from 100 million medical images with optional supervision", JOURNAL OF MEDICAL IMAGING, SOCIETY OF PHOTO-OPTICAL INSTRUMENTATION ENGINEERS, 1000 20TH ST. BELLINGHAM WA 98225-6705 USA, vol. 9, no. 6, 30 November 2022 (2022-11-30), page 64503, XP060172039, ISSN: 2329-4302, DOI: 10.1117/1.JMI.9.6.064503 [retrieved on 2022-11-30] * abstract; figures 1-4, 9, 11 * * sections 1-4 * | 1-20 | INV. G06N3/045 G06N3/084 G06N3/096<br><br>ADD. G06N3/0464 |
| A | WEI-CHIEN WANG ET AL: "A Review of Predictive and Contrastive Self-supervised Learning for Medical Images", ARXIV.ORG, CORNELL UNIVERSITY LIBRARY, 201 OLIN LIBRARY CORNELL UNIVERSITY ITHACA, NY 14853, 10 February 2023 (2023-02-10), XP091435274, * the whole document * | 1-20 | |
| A | YANBEI CHEN ET AL: "Semi-Supervised and Unsupervised Deep Visual Learning: A Survey", ARXIV.ORG, CORNELL UNIVERSITY LIBRARY, 201 OLIN LIBRARY CORNELL UNIVERSITY ITHACA, NY 14853, 24 August 2022 (2022-08-24), XP091301417, * the whole document *<br><br>-/-- | 1-20 | |

TECHNICAL FIELDS SEARCHED (IPC)

G06N
G06T
G16H

The present search report has been drawn up for all claims

| Place of search | Date of completion of the search | Examiner |
|---|---|---|
| The Hague | 18 July 2023 | Cilia, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (IPC) |
| A | KWASIGROCH ARKADIUSZ ET AL: "Self-Supervised Learning to Increase the Performance of Skin Lesion Classification", ELECTRONICS, vol. 9, no. 11, 17 November 2020 (2020-11-17), page 1930, XP093064577, DOI: 10.3390/electronics9111930 * the whole document * ----- | 1-20 | |
| A | UTKARSH OJHA ET AL: "What Knowledge Gets Distilled in Knowledge Distillation?", ARXIV.ORG, CORNELL UNIVERSITY LIBRARY, 201 OLIN LIBRARY CORNELL UNIVERSITY ITHACA, NY 14853, 3 October 2022 (2022-10-03), XP091331652, * the whole document * ----- | 1-20 | |
| | | | TECHNICAL FIELDS SEARCHED (IPC) |

The present search report has been drawn up for all claims

| Place of search | Date of completion of the search | Examiner |
|---|---|---|
| The Hague | 18 July 2023 | Cilia, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2